Europäisches Patentamt

European Patent Office

Office européen des brevets

(11) Publication number: **0 124 366 B1**

# EUROPEAN PATENT SPECIFICATION

(45) Date of publication of patent specification: **30.10.91**  (51) Int. Cl.⁵: **G01N 33/543**, G01N 33/535, G01N 33/545, G01N 33/58

(21) Application number: **84302853.1**

(22) Date of filing: **27.04.84**

(54) Method of measuring biological ligands.

(30) Priority: **30.04.83 JP 77255/83**

(43) Date of publication of application:
**07.11.84 Bulletin 84/45**

(45) Publication of the grant of the patent:
**30.10.91 Bulletin 91/44**

(84) Designated Contracting States:
**CH DE FR GB IT LI NL**

(56) References cited:
**EP-A- 0 071 976
EP-A- 0 094 777
GB-A- 2 013 211
US-A- 4 228 237**

(73) Proprietor: **FUJIREBIO KABUSHIKI KAISHA also trading as FUJIREBIO INC.
6-7, Shimoochiai 4-chome Shinjuku-ku Tokyo 161(JP)**

(72) Inventor: **Kasahara, Yasushi
310, 4-4, Nagayama 3-chome
Tama-shi Tokyo(JP)**
Inventor: **Ashihara, Yoshihiro
2-402, Fuchudanchi 28-1, Harumicho
1-chome
Fuchu-shi Tokyo(JP)**
Inventor: **Suzuki, Hiromasa
36-12, Shimizucho
Itabashi-ku Tokyo(JP)**

(74) Representative: **Silverman, Warren et al
HASELTINE LAKE & CO. Hazlitt House 28
Southampton Buildings Chancery Lane
London WC2A 1AT(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 124 366 B1

## Description

This invention relates to a method of measuring a biological ligand, for example, a medicinal substance or a trace compound resulting from one of various diseases and to be found in a humoral fluid such as blood serum or urine.

The measurement of the concentration in a body (or humoral) fluid, in particular the blood, of a drug such as digoxin and theophylline, administered to a patient, is important for the appropriate treatment, and the detection in the blood of a subject of trace compounds formed with various diseases such as cancer is important for the early diagnosis of diseases.

Accordingly, various methods have been developed to detect these trace components in blood. Among these, enzyme immunoassay has come to be widely employed because of its high sensitivity, specificity and capability for rapid treatment of a large number of samples. However, the sensitivity is not sufficient with conventional enzyme immunoassay, and it is not easy to determine concentrations exactly because of variations in results which may arise owing to complicated washing procedures and transferring of tubes.

It is an object of this invention to provide a method of measuring a biological ligand which allows exact concentrations to be measured with high sensitivity, specificity and capability for use on a large number of samples concurrently.

According to the present invention, there is provided a method of measuring a biological ligand, characterised by allowing there to coexist in an aqueous medium:

(A) a biologically active composition comprising firstly an immobilised antibody phase whose antibody is capable of reacting with a ligand (1) to be measured or an immobilised phase of a biological ligand (2) which is capable of reacting with said antibody and secondly an immobilised biotinyl enzyme phase or immobilised biotinyl enzyme inhibitor phase,

(B) a water-soluble material combination of whichever of said ligand (2) or said antibody on the one hand and a biotinyl enzyme inhibitor or a biotinyl enzyme on the other hand are absent from the biologically active composition, the biotinyl enzyme inhibitor then being one which is capable of reacting with the biotinyl enzyme of the biologically active composition or the biotinyl enzyme then being one which is capable of reacting with the biotinyl enzyme inhibitor of the biologically active composition, and

(C) the ligand (1) to be measured,

and measuring the residual biotinyl enzyme activity or the residual biotinyl enzyme inhibitory activity of said biologically active composition or said aqueous solution and utilising the result as a measure of the ligand.

This invention is a development of the enzyme immunoassay method for the determination of antigen or antibody of our copending European Patent Application No.83302591.9 (published on 23.11.83 under number 0094777) where a biologically active composition comprising an immobilised antigen or antibody phase and an immobilised enzyme, enzyme inhibitor or enzyme activator phase is employed. In particular, the method of the present invention is a development of such method which generally uses the biotin-avidin reaction. This method possesses higher sensitivity and Simplicity than hitherto used methods and utilises the small coupling constant of biotin and avidin.

The subject to be measured by the method of this invention is a ligand (1). A ligand in the biological or medical field is a substance having one or more antigenic determinants, and such ligands include hormones derived from various endocrine glands and exemplified by insulin, TSH and thyroglobulin; plasma proteins for example immunoglobulin, albumin and ferritin; viral antigens for example HB antigen, bacteria, $\alpha$-fetoprotein, and carcinoembryonic antigens. This ligand (1) may be also a hapten and a first antibody in the double antibody method. The ligand (1) includes, for example, the material combination of the target antigen to be measured and a first antibody in the case of the double antibody method.

The biologically active composition comprises an immobilised antibody phase or immobilised phase containing a ligand (2) capable of reacting with such antibody and an immobilised biotinyl enzyme or biotinyl enzyme inhibitor phase.

In carrying out the method of this invention, the aforementioned antibody reacts with the ligand (1), and the ligand (2) reacts with this antibody. Thus, however the antibody and ligand (2) are provided, at least one antigenic determinant should be common to the two ligands, and the antibody should be the antibody against this common antigenic determinant. The antibody may be an immunoglobulin fragment such as F-(ab')$_2$, Fab' and Fab. All antigenic determinants for ligand (2) may be identical to those of ligand (1), and accordingly, ligand (2) may be identical with ligand (1).

The antibody may be produced by a known method for producing an antibody. For example, the ligand (1), the ligand (2) or a material combination of either of these ligands and a proteinaceous material is injected once or several times into the subcutaneous region of the back, foot pad or femoral muscle of a warm-blooded animal, such as rabbit, goat, horse, guinea pig and chicken, in an amount of 0.3 to 2 mg per

2

kg, together with an adjuvant. The antibody is then produced in a body fluid such as serum. This fluid may be used as such as a source of the antibody. However, the antibody is preferably separated by the conventional isolation method used for immunoglobulin.

On the other hand, the antibody may be produced as a monoclonal antibody. In this case, one of the above antigens is injected several times into the abdominal cavity of a mouse together with an adjuvant, and its spleen is excised. A spleen cell is fused with a mouse myeloma cell by a conventional method, for example using polyethylene glycol. The hybridoma thus obtained is cultured and cloned, and a cell capable of producing the target antibody is obtained. This cell is injected into the abdominal cavity of a mouse, and multiplied. Then, ascites are collected, and the target antibody is separated from the ascites.

A biotinyl enzyme has a biotinyl group at its active site, and is, for example, propionyl CoA carboxylase, acetyl CoA carboxylase, pyruvate carboxylase, methylmalonyl CoA carboxylase, methylmalonyl CoA transcarboxylase or methylcrotonyl CoA carboxylase.

Biotinyl enzyme inhibitors include avidin, streptoavidin and their derivatives capable of binding to biotin. Such derivatives may be conventional derivative-types, obtainable by reaction with chemical modifiers, and include acetylated derivatives. The coupling constants of the derivatives are preferably small.

Since the ligand (2) or the antibody on the one hand and the biotinyl enzyme or the biotinyl enzyme inhibitor on the other hand can be respectively immobilised in the biologically active composition, there are four kinds of combination which may be present, namely (a) ligand (2) and biotinyl enzyme, (b) ligand (2) and biotinyl enzyme inhibitor, (c) antibody and biotinyl enzyme, and (d) antibody and biotinyl enzyme inhibitor.

Such a biologically active composition is distinguished by the separation of the immobilised ligand (2) phase or antibody phase on the one hand from the immobilised biotinyl enzyme phase or biotinyl enzyme inhibitor phase. This phase separation means that when the material combination of a ligand (2) or an antibody on the one hand and biotinyl enzyme or biotinyl enzyme inhibitor to be described later herein reacts and combines with one immobilised phase, this material combination cannot combine with the other immobilised phase. However, of course, the material combination may react with both immobilised phases at the boundary regions thereof.

Turning to the specific materials to be employed, the biologically active composition may include a single polymer material on which a ligand (2) or an antibody on the one hand and a biotinyl enzyme or a biotinyl enzyme inhibitor on the other hand are bound at different locations. Alternatively, there may be two polymer materials which are bonded to each other, with ligand (2) or an antibody being immobilised on one polymer material and a biotinyl enzyme or a biotinyl enzyme inhibitor being immobilsed on the other. In addition, where relatively large polymer particles, for example, having a diameter of more than 3 mm can be considered since the contacting area between the separate particles is likely to be small in comparison with the total surface area, the above biologically active composition may consist of a mixture of two particle groups with the particles belonging to one group having a ligand (2) or an antibody immobilised thereon and the particles belonging to the other group having a biotinyl enzyme or a biotinyl enzyme inhibitor immobilised thereon. In such a case, the particles of one group may be replaced by a polymer tube in which the particles of the other group are located.

The biologically active composition is prepared as follows. When it is to comprise a polymer material where the biologically active substances are immobilised at different locations thereon, the polymer material may be a block-copolymer or a graft-copolymer. In this case, the ligand (2) or antibody on the one hand and biotinyl enzyme or biotinyl enzyme inhibitor on the other hand are immobilised at separate locations by using different functional groups in such copolymers. $-NH_2$, $-COOH$, $-CHO$, $-OH$ and $-SH$ may be used as the functional groups. Should there be used a ligand (2) and/or a biotinyl enzyme inhibitor which does not lose activity through the copolymerisation reaction, the immobilisation may be carried out prior to the copolymerisation. In such case, the functional groups may be identical.

When employing the two polymer materials which are to be bound to one another, the immobilisations may be carried out before or after the bonding. The bonding may be carried out by a welding method or by use of an adhesive.

A biologically active composition which is a single body may be, for example, a sphere, a disc, a cube or a tube.

The immobilisation may be carried out by following any convenient method. A biologically active substance which is a protein may be immobilised by using a method for immobilising biologically active protein such as enzyme. Such methods include use of covalent bonding as in the diazotization method, the peptide synthesis method and the alkylation method, ionic bond formation and physical adsorption. When material other than protein material is to be bound it is necessary to consider the functional groups of the biologically active substance and those of the carrier material. An immobilised ligand (2) may be the reaction product of a ligand and a first antibody in which case the first antibody is allowed to react with the ligand covalently bound to a carrier material and then the reaction product is further allowed to react with a second antibody.

The quantitative relationship between the ligand (2) or the antibody on the one hand and the biotinyl enzyme or the biotinyl enzyme inhibitor on the other hand, both of which are immobilised on the biologically active composition will depend on their reactivity, the sensitivity of the measuring apparatus, etc.

The essential constituents of the material combination are firstly an antibody capable of reacting with the ligand (1) and with the ligand (2) immobilised on the biologically active composition or alternatively the ligand (2) capable of reacting with the antibody of the biologically active composition and secondly a biotinyl enzyme inhibitor capable of reacting with the biotinyl enzyme of the biologically active composition or a biotinyl enzyme capable of reacting with the biotinyl enzyme inhibitor of the biologically active composition. Accordingly, there are four possible combinations, namely (A) ligand (2) and biotinyl enzyme, (B) ligand (2) and biotinyl enzyme inhibitor, (C) antibody and biotinyl enzyme, and (D) antibody and biotinyl enzyme inhibitor. However, since these constituents have to react with the corresponding immobilised phase of the biologically active composition, their choice will depend on the character of the biologically active composition. The material combination needs to be water-soluble because its components need to be able to react with the active components of the biologically active composition.

When both components of the material combination are proteinaceous, such a material combination may be produced by the cross-linking method or by a peptide synthesis method. In the case that one or both materials is not a protein, a suitable method for producing the material combination will depend upon the functional groups of the materials to be combined. The molar ratio of the material combination is not limited to 1:1, and a suitable ratio will generally depend on the measuring conditions.

The order of bringing into contact with each other, in aqueous solution, the biologically active composition, the material combination and the ligand (1) to be measured is arbitrary. These materials do not need to be in contact for the entire course of one determination, and such contact may be for only a short time. For example, a ligand (1) to be measured and the material combination of a ligand (2) and a biotinyl enzyme may be allowed to react with an antibody immobilised on a tube and then the remaining material combination is scavenged by adding a biotinyl enzyme inhibitor immobilised on a bead.

The aqueous solution employed is preferably kept at a suitable pH for the reaction of the ligands and the antibody and for the reaction of the biotinyl enzyme and the biotinyl enzyme inhibitor, and a buffer solution such as a phosphate buffer solution, a borate buffer solution or a tris buffer solution is preferably used for the purpose. The pH of the buffer depends on the identity of the ligands, the antibody, the biotinyl enzyme and the biotinyl enzyme inhibitor, but it is usually in the range of pH 5 to 8. The temperature of the aqueous solution may also be varied according to the character of the ligands, the antibody, the biotinyl enzyme and the biotinyl enzyme inhibitor, and it is usually in the range of from 15 to 45°C.

After the reaction, the biotinyl enzyme activity as such, the biotinyl enzyme activity of the biologically active composition or the biotinyl enzyme activity of the aqueous solution is measured. The measurement is carried out by any convenient method. For example, in the case of propionyl CoA carboxylase as biotinyl enzyme, ADP produced during the reaction is allowed to react in the presence of pyruvate kinase and lactate dehydrogenase, and the decrease of NADH is colourimetrically determined. In the case of methylmalonyl CoA transcarboxylase, oxalacetic acid produced is converted to malic acid in the presence of malate dehydrogenase, and the decrease of NADH is colourimetrically measured. When using propionyl CoA carboxylase, ATP produced by a reverse reaction is allowed to react in the presence of luciferase and luciferin, and thereby bioluminescence is produced which can be measured by using a photon counter even for determination of ATP at a concentration lower than $10^{-15}$ M. The method of this invention is thus suitable for determining an extremely small amount of the ligand (1).

In summary thus, the method of the invention enables a ligand (1) to be determined to a high sensitivity. For example, when using a biotinyl enzyme and avidin, a ligand (1) present in an amount of $10^{-11}$ gram can be determined. Furthermore, the operation of the present method is simple, and for example, a separation process for a bound reagent and a free reagent after contacting of all reagents is not necessary. Hence, a ligand (1) can easily and inexpensively be determined.

The following examples illustrate this invention.

EXAMPLE 1

(1) Preparation of material combination

5 mg of avidin were dissolved in 1 ml of 0.1 M phosphate buffer solution of pH 6.3, and 100 µl of 2 mg/ml 4-maleimidomethyl cyclohexane-1-carboxylic acid succinimide ester (CHM) solution in dioxane were added, and allowed to react at ambient temperature for one hour. The reaction solution obtained was introduced into a column of Sephadex® G-25 (0.1 M phosphate buffer solution -1mM EDTA pH 6.5), and gel filtration was carried out. The protein fractions were collected as CHM induced avidin fractions.

Goat anti-human α-fetoprotein (AFP) antibody was purified by using an AFP affinity column, and 10 mg of the purified IgG were dissolved in 1.0 ml of 0.1 M phosphate buffer solution - 1 mM EDTA of pH 6.0. 0.1 ml of 0.1 M 2-mercaptoethylamine solution was added to this solution, and the mixture of solutions was allowed to react at 37°C for 90 minutes. The reaction solution obtained was passed through a column of Sephadex® G-25, and the void fractions were collected. The above CHM induced avidin was added to this void fraction, and the pH of the mixture was adjusted to 6.8. The mixture was allowed to react at 4°C for 24 hours, and the reaction solution was concentrated by using PEG-20,000. The concentrate obtained was separated by gel filtration using Sephacryl® S-300, and about 7 mg of a material combination of anti-AFP IgG-avidin were obtained.

(2) Preparation of Biologically Active Composition

A spherical nylon bead 4 mm in diameter was treated with 3N HCl for 24 hours to liberate its amino groups. The treated nylon bead was washed with water and immersed in 0.1 M phosphate buffer solution - 5 mM EDTA pH 7.5. A 10 mg/ml solution of S-acetylmercaptosuccinic anhydride in dimethyl sulphoxide whose volume was one tenth that of the above buffer solution was added, and allowed to react at 37°C for 3 hours. 1 M hydroxylamine solution of pH 7.5 and whose volume was one tenth of that of the above reaction solution was added to this, and allowed to react at 37°C for 1 hour. Then, the nylon bead was washed with the above buffer solution.

5 mg of CHM induced propionyl CoA carboxylase prepared in the same manner as the avidin of step (1) were dissolved in 0.1 M phosphate buffer solution - 1 mM EDTA of pH 6.0, and this solution was allowed to contact the washed nylon bead. The solution was adjusted to pH 7.0, and allowed to react at 4°C overnight. Then, the nylon bead was washed with the above buffer solution of pH 7.5, and was kept as an immobilised propionyl CoA carboxylase bead in the above buffer solution containing 1% BSA (bovine serum albumin).

A spherical polystyrene bead 3.2 mm in diameter washed with water was immersed overnight at 4°C in a phosphate buffer solution of pH 7.5 containing AFP ($OD_{280}$ = 0.1). In this way, AFP was adsorbed on the polystyrene bead. This polystyrene bead was washed well with a phosphate buffer solution of pH 7.5, and was kept as an immobilised AFP bead in the above buffer solution containing 1% by weight BSA.

(3) Measurement of AFP

One immobilised propionyl CoA carboxylase bead and one immobilised AFP bead were placed in each of a series of test tubes, and 800 μl of 50 mM tris buffer solution - 0.14 M NaCl - 0.2% BSA of pH 7.8 were added to each test tube.

800 ng/ml AFP solution was prepared, and the solution was diluted to produce 4n diluted solution series.

Each 100 μl of the diluted solution and then 100 μl of the anti AFP IgG - avidin material combination of step (1) were added to each test tube, and allowed to stand at ambient temperature for one hour.

Subsequently, 1 ml of an enzyme substrate solution (containing 0.1 M KCl - 50 mM $KHCO_3$ - 4 mM $MgCl_2$ - 2 mM reduced glutathione - 2 mM ATP - 1 mM phosphoenolpyruvic acid - 2,500 U/l pyruvate kinase - 3,500 U/l lactate dehydrogenase - 0.15 mM NADH - 2 mM propionyl CoA) was added to each test tube, and the absorbance at 340 nm of the reaction solution at 37°C was measured to determine the relation between the AFP concentration and the activity of propionyl CoA carboxylase.

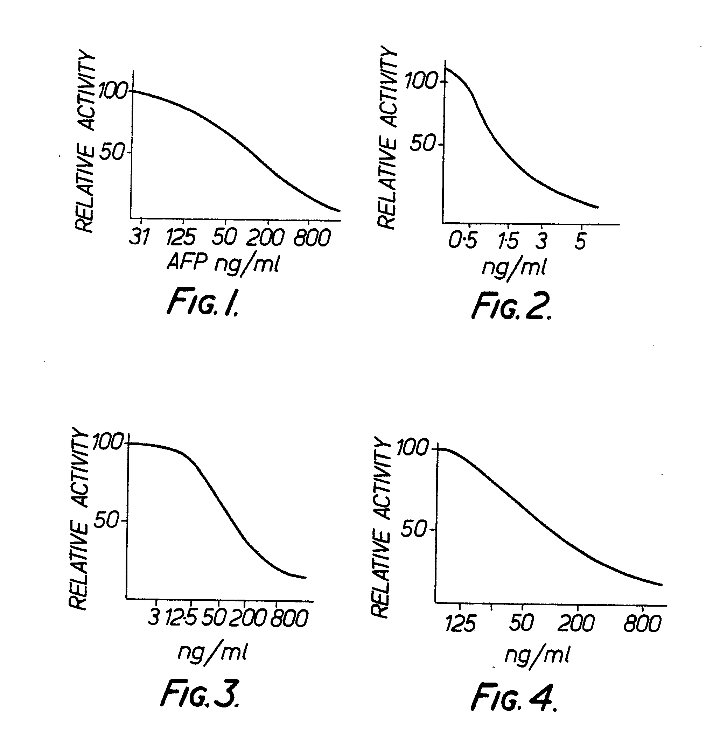

The results obtained were plotted and are shown in Figure 1 of the accompanying drawing.

The AFP concentrations of various human sera were then determined. Each serum sample was allowed to react with avidin immobilised on glass beads for 30 minutes, and the supernatant obtained was then diluted 10 times. Using 100 μl of the diluted supernatant in each case, measurements were carried out in the same manner as above. The AFP concentrations of each serum were determined by using the curve of Figure 1 as a calibration curve. The AFP concentrations of the same sera were also measured by the conventional radioimmunoassay (RIA) methods. The results obtained are compared in the following Table 1.

Table 1

| AFP Concentration (mg/ml) | | |
| --- | --- | --- |
| Serum | The method of the invention | RIA method |
| A1 | 15.8 | 17.5 |
| A2 | 450 | 432 |
| A3 | 142 | 158 |
| A4 | 6.3 | 6.9 |
| A5 | 748 | 780 |
| A6 | 210 | 200 |

## EXAMPLE 2

(1) preparation of material combination

5 mg of pyruvate carboxylase were dissolved in 1 ml of 0.1 M phosphate buffer solution of pH 6.3, and 100 $\mu$l of 2 mg/ml CHM dioxane solution were added. The mixture obtained was allowed to react at ambient temperature for 90 minutes. Then, the resulting reaction solution was separated by gel filtration using a column of Sephadex G-25 which had been previously equilibrated with 0.1 M phosphate buffer solution - 1 mM EDTA pH 6.5, and the void fractions were collected. The fractions were concentrated to 1 ml by using PEG 20,000 to obtain CHM induced pyruvate carboxylase.

5 mg of anti-digoxin rabbit IgG were dissolved in 0.1 M phosphate buffer solution - 5 mM EDTA of pH 7.5, and 100 $\mu$l of 9 mg/ml acetylmercaptosuccinic anhydride dimethyl sulphoxide solution were added. The mixture was allowed to react at 37°C for one hour. 110 $\mu$l of 1 M hydroxylamine solution were added to the reaction mixture, and allowed to react at 37°C for 30 minutes. The reaction solution was then treated by gel filtration using a Sephadex G-25 column as above, and the void fractions were collected. The CHM induced pyruvate carboxylase was added to the void fractions, and they were allowed to stand at 4°C overnight. The reaction solution was separated by gel filtration using Sephacryl S-300, and the fractions of the material combination of anti-digoxin IgG - pyruvate carboxylase (1:1 ratio) were collected.

(2) Preparation of Biologically Active Composition

2 ml of 100 $\mu$g/ml avidin - 20 mM PBS solution of pH 7.8 and a polystyrene tube (8 mm x 50 cm) were brought into contact and allowed to stand at 4°C for 16 hours. This tube was washed 4 times with a saline solution. The tube was brought into contact with 5% by weight aqueous bovine serum albumin BSA solution and allowed to stand at ambient temperature for 3 hours to obtain an avidin-sensitised tube.

Next, digoxin was oxidised using NaIO$_4$ and allowed to combine with BSA. 20 mg of the BSA - digoxin material combination were dissolved in 100 ml of 0.02 M phosphate buffer solution of pH 8.0, and 60 polystyrene beads, 4 mm in diameter, were added. The beads were allowed to stand at 4°C for 16 hours to obtain digoxin-sensitised polystyrene beads.

(3) Measurement of Digoxin

One digoxin-sensitised bead was added to each avidin-sensitised tube, and 400 $\mu$l of 20 mM tris buffer solution - 0.14 M NaCl - 0.2% BSA of pH 8.0 were added to each tube. 100 $\mu$l of a diluted digoxin solution (0-5 ng/ml) and 100 $\mu$l of the material combination of anti-digoxin IgG - pyruvate carboxylase prepared in step (1) were added to each tube which was then kept at 30°C for 1 hour.

Subsequently, 1 ml of an enzyme substrate solution (containing 0.1 M KCl - 50 mM KHCO$_3$ - 4 mM MgCl$_2$ - 2 mM ATP - 2 mM reduced glutathione - 4 mM pyruvic acid - 0.23 mM NADH - 3000 U/l malate dehydrogenase, pH 7.5) was added to each tube, and the decreasing rate of the absorbance at 340 nm at 37°C was measured. The relation between the relative activity of pyruvate carboxylase and the concentration of digoxin thus obtained was plotted and is shown in Figure 2 of the accompanying drawings.

The digoxin concentrations of various human sera were then determined. Each serum sample was allowed to react with avidin immobilised on glass beads for 30 minutes, and the supernatant obtained was then diluted 10 times. Using 100 $\mu$l of the diluted supernatant in each case, measurements were carried out

6

in the same manner as above using Figure 2 as a calibration curve. The digoxin concentrations of the sera were also measured by the RIA method. The results obtained are shown in the following Table 2.

Table 2

| Digoxin Concentration (ng/ml) | | |
|---|---|---|
| Serum | The method of the invention | RIA method |
| B1 | 1.5 | 1.3 |
| B2 | 2.2 | 2.6 |
| B3 | 4.1 | 4.0 |
| B4 | 0.8 | 1.1 |
| B5 | 1.1 | 1.3 |

EXAMPLE 3

(1) Preparation of material combination

5 mg of anti-ferritin goat IgG were dissolved in 1 ml of 0.1 M phosphate buffer solution - 1 mM EDTA of pH 6.3, and 100 μl of 2 mg/ml CHM dioxane solution were added to this. The mixture was allowed to react at ambient temperature for 90 minutes. The reaction mixture produced was separated by gel filtration using Sephadex® G-25, and the void fractions were collected.

5 mg of ferritin and 1 mg of avidin were dissolved in 1 ml of 0.1 M phosphate buffer solution of pH 6.8, and 100 μl of 1% glutaraldehyde aqueous solution were added to this, and allowed to react at 30°C for 3 hours. The reaction mixture was separated by gel filtration using Sephacryl S-300, and the fractions of the material combination of ferritin and avidin (1:1 molar ratio) were collected. The fractions were lyophilised to obtain 3 mg of the target material combination of ferritin-avidin.

(2) Preparation of Biologically Active Composition

A polystyrene carrier plate was prepared by the known polymer blend method as follows:

10 mg each of poly-L-cysteine and poly-L-lysine were dissolved in a dimethyl sulphoxide - ether mixture solvent, and applied to a polystyrene plate 5 cm square. The solvent was then evaporated under reduced pressure. A micro phase separation between the poly-L-cysteine layer and the poly-L-lysine layer formed on the surface of the plate owing to the difference in their condensation energies. The SH group content of the plate coating was measured by the dithiopyridone method and the amino group content was measured by the ninhydrin method, and the ratio of the SH groups to amino groups was found to be about 1:1. This polystyrene plate was cut into squares of 0.8 cm sides, each to be used as a carrier plate subsequently.

Each carrier plate was immersed in acetyl CoA carboxylase aqueous solution to which water-soluble carbodiimide had been added until its concentration was 100 mg/10 ml. The mixture with the carrier plate in it was adjusted to pH 5.5 by using 0.1M(N) NaOH, and allowed to stand at 4°C for 16 hours. Then, the plate was well washed with 20 mM phosphate buffered saline solution of pH 7.0.

This plate was then immersed in 0.1 M phosphate buffer solution - 1 mM EDTA of pH 6.5, and CHM induced anti-ferritin goat IgG prepared in step (1) was added to be in a concentration of 2 mg/ml. The mixture obtained was allowed to react at 30°C for 2 hours, and the plate was well washed with 20 mM PBS of pH 7.0 to obtain the required biologically active composition.

(3) Measurement of Ferritin

Each biologically active plate prepared in step (2) was placed in a glass test tube, and 400 μl of 20 mM tris buffer solution - 0.14 M NaCl - 0.2% BSA of pH 8.0 were added. 50 μl quantities of ferritin solution of different concentrations and 50 μl of the solution of the material combination prepared in step (1) were added to the test tube, and incubated at 30°C for 1 hour. 1 ml of a substrate solution (containing 0.1 M KCl - $10^{-3}$M acetyl CoA - 0.3 M KHCO$_3$- 3 mM ATP - 0.23 mM NADH - 2 mM reduced glutathione - 1.5 mM phosphoenolpyruvic acid - 2,500 U/l pyruvate kinase - 3,500 U/l lactate dehydrogenase, pH 7.8) was added to each test tube, and the decreasing rate of the absorbance at 340 nm at 37°C was measured. The relation between the relative activity of acetyl CoA carboxylase and the known concentration of ferritin thus obtained was plotted and is shown in Figure 3 of the accompanying drawings.

The ferritin concentrations of various human sera were then determined. Each serum sample was allowed to react with avidin immobilised on glass beads for 30 minutes, and the supernatant was diluted 10 times. Using 100 $\mu$l of the diluted supernatant in each case, measurements were carried out in the same manner as above. The results obtained are shown in the following Table 3. For the purpose of comparison, the ferritin concentrations of the same sera were measured by the conventional enzyme immunoassay (EIA) method. The results are also shown in Table 3.

Table 3

| | Ferritin Concentration (ng/ml) | |
|---|---|---|
| Serum | The method of the invention | EIA method |
| C1 | 19 | 21 |
| C2 | 169 | 176 |
| C3 | 145 | 146 |
| C4 | 38 | 33 |
| C5 | 395 | 400 |

## EXAMPLE 4

### (1) Preparation of material combination

5 mg of ferritin and 10 mg of propionyl CoA carboxylase were dissolved in 1 ml of 0.1 M phosphate buffer solution pH 6.8, and 100 $\mu$l of 1% by weight glutaraldehyde aqueous solution were added. The mixture was allowed to react at 30°C for 4 hours. The reaction mixture was separated by gel filtration using 20 mM phosphate buffered (pH 7.0) Sepharose 4B, and the fractions of the material combination were collected. In this way, 5 mg of the target material combination in which the ferritin and propionyl CoA carboxylase were present in a 1:1 molar ratio were obtained.

### (2) Preparation of Biologically Active Composition

A biologically active composition was prepared in which anti-ferritin goat IgG and avidin were separately immobilised on a carrier plate according to the procedure of step (2) of Example 3.

### (3) Measurement of Ferritin

Specimens of the biologically active composition prepared in step (2) were placed in test tubes, and 800 $\mu$l of 50 mM tris buffer solution - 0.14 M NaCl - 0.2% BSA of pH 7.8 were added to each test tube. A ferritin solution of 1,000 ng/ml was diluted, and 4n diluted solution series was prepared. 100 $\mu$l of the diluted solution and 100 $\mu$l of the material combination prepared in step (1) were added to each test tube, and allowed to stand at ambient temperature for 1 hour.

1 ml of the same enzyme substrate solution as employed in Example 1 was added to each test tube, and the decreasing rate of the absorbance at 340 nm at 37°C was measured to determine the relation between the known ferritin concentration and the relative activity of propionyl CoA carboxylase. The results obtained were plotted as a calibration curve which is shown in Figure 4 of the accompanying drawings.

The ferritin concentrations of various human sera were then determined. Each serum sample was allowed to react with immobilised avidin glass beads for 30 minutes, and the supernatant was diluted 10 times. Using 100 $\mu$l of the diluted supernatant in each case measurements were carried out in the same manner as above. The results obtained are shown in the following Table 4. For the purpose of comparison, the ferritin concentrations of the same sera were measured by the conventional EIA method. The results are also shown in the Table 4.

EP 0 124 366 B1

Table 4

| Ferritin Concentration (ng/ml) | | |
|---|---|---|
| Serum | The method of the invention | EIA method |
| D1 | 20 | 21 |
| D2 | 180 | 176 |
| D3 | 150 | 146 |
| D4 | 35 | 33 |
| D5 | 391 | 400 |

## Claims

1. A method of measuring a biological ligand, characterised by allowing there to coexist in an aqueous medium:

    (A) a biologically active composition comprising firstly an immobilised antibody phase whose antibody is capable of reacting with a ligand (1) to be measured or an immobilised phase of a biological ligand (2) which is capable of reacting with said antibody and secondly an immobilised biotinyl enzyme phase or immobilised biotinyl enzyme inhibitor phase,

    (B) a water-soluble material combination of whichever of said ligand (2) or said antibody on the one hand and a biotinyl enzyme inhibitor or a biotinyl enzyme on the other hand are absent from the biologically active composition, the biotinyl enzyme inhibitor then being one which is capable of reacting with the biotinyl enzyme of the biologically active composition or the biotinyl enzyme then being one which is capable of reacting with the biotinyl enzyme inhibitor of the biologically active composition, and

    (C) the ligand (1) to be measured,

    and measuring the residual biotinyl enzyme activity or the residual biotinyl enzyme inhibitory activity of said biologically active composition or said aqueous solution and utilising the result as a measure of the ligand.

2. A method as claimed in claim 1, wherein said ligand (1) and said ligand (2) are selected from hormones derived from an endocrine gland, plasma proteins, viral antigens, bacteria, α -fetoprotein, carcinoembyronic antigens, haptens and first antibodies in the double antibody method.

3. A method as claimed in claim 1 or 2, wherein said ligand (1) and said ligand (2) are the same substance.

4. A method as claimed in any one of the preceding claims, wherein said antibody is a fragment of immunoglobulin.

5. A method as claimed in any one of claims 1 to 3, wherein said antibody is produced from a warm-blooded animal.

6. A method as claimed in any one of claims 1 to 3, wherein said antibody is a monoclonal antibody.

7. A method as claimed in any one of the preceding claims, wherein said biotinyl enzyme is propionyl CoA carboxylase, acetyl CoA carboxylase, pyruvate carboxylase, methylmalonyl CoA carboxylase, methylmalonyl CoA transcarboxylase or methylcrotonyl CoA carboxylase.

8. A method as claimed in any one of the preceding claims, wherein said biotinyl enzyme inhibitor is avidin, streptoavidin or a derivative thereof capable of binding to biotin.

9. A method as claimed in any one of the preceding claims, wherein the carrier material of said biologiclly active composition is a single block-copolymer or graft-copolymer, two polymer materials which are bound to each other or two separate bodies for immobilising respective biologically active components.

10. A method as claimed in any one of the preceding claims, wherein said aqueous solution is at a pH 5 to 8 and has a temperature of from 15 to 45°C.

11. A method as claimed in claim 10, wherein said aqueous solution is an aqueous buffer solution.

12. A method as claimed in any one of the preceding claims, wherein, in measuring the biotinyl enzyme activity or the biotinyl enzyme inhibitory activity, a reaction is carried out which produces bioluminescence which is measured quantitatively using a photon counter.

**Revendications**

1. Procédé de mesure d'un ligand biologique, caractérisé en ce qu'on laisse en contact, dans un milieu aqueux:

   (A) une composition biologiquement active comprenant, premièrement, une phase à anticorps immobilisé, dont l'anticorps est capable de réagir avec le ligand (1) à mesurer, ou une phase à ligand biologique (2) immobilisé qui est capable de réagir avec ledit anticorps, et, deuxièmement, une phase à enzyme biotinylée immobilisée ou une phase à inhibiteur d'enzyme biotinylée immobilisé,

   (B) une combinaison hydrosoluble de celles des substances, ledit ligand (2) ou ledit anticorps d'une part, et l'inhibiteur d'enzyme biotinylée ou l'enzyme biotinylée d'autre part, qui sont absentes de la composition biologiquement active, l'inhibiteur d'enzyme biotinylée étant alors un inhibiteur capable de réagir avec l'enzyme biotinylée de la composition biologiquement active, ou bien l'enzyme biotinylée étant alors une enzyme capable de réagir avec l'inhibiteur d'enzyme biotinylée de la composition biologiquement active, et

   (C) le ligand (1) à mesurer,

   et en ce que l'on mesure l'activité résiduelle d'enzyme biotinylée ou l'activité résiduelle d'inhibiteur d'enzyme biotinylée de ladite composition biologiquement active ou de ladite solution aqueuse, et en ce que l'on utilise le résultat comme mesure du ligand.

2. Procédé tel que revendiqué dans la revendication 1, dans lequel ledit ligand (1) et ledit ligand (2) sont choisis parmi des hormones provenant d'une glande endocrine, des protéines plasmatiques, des antigènes viraux, des bactéries, l'α-foetoprotéine, des antigènes carcino-embryonnaires, des haptènes et les premiers anticorps dans une méthode à double anticorps.

3. Procédé tel que revendiqué dans l'une des revendications 1 ou 2, dans lequel ledit ligand (1) et ledit ligand (2) sont la même substance.

4. Procédé tel que revendiqué dans l'une quelconque des revendications précédentes, dans lequel ledit anticorps est un fragment d'immunoglobuline.

5. Procédé tel que revendiqué dans l'une quelconque des revendications 1 à 3, dans lequel ledit anticorps est produit à partir d'un animal à sang chaud.

6. Procédé tel que revendiqué dans l'une quelconque des revendications 1 à 3, dans lequel ledit anticorps est un anticorps monoclonal.

7. Procédé tel que revendiqué dans l'une quelconque des revendications précédentes, dans lequel ladite enzyme biotinylée est la propionyl-CoA carboxylase, l'acétyl-CoA carboxylase, la pyruvate carboxylase, la méthylmalonyl-CoA carboxylase, la méthylmalonyl-CoA transcarboxylase ou la méthylcrotonyl-CoA transcarboxylase.

8. Procédé tel que revendiqué dans l'une quelconque des revendications précédentes, dans lequel ledit inhibiteur d'enzyme biotinylée est l'avidine, la streptoavidine ou un de leurs dérivés capables de se lier à la biotine.

9. Procédé tel que revendiqué dans l'une quelconque des revendications précédentes, dans lequel le matériau support de ladite composition biologiquement active est un copolymère séquencé ou un copolymère greffé unique, deux matériaux polymères liés l'un à l'autre ou deux corps séparés pour

EP 0 124 366 B1

immobiliser les composants biologiquement actifs respectifs.

10. Procédé tel que revendiqué dans l'une quelconque des revendications précédentes, dans lequel ladite solution aqueuse présente un pH de 5 à 8 et une température de 15 à 45° C.

11. Procédé tel que revendiqué dans la revendication 10, dans lequel ladite solution aqueuse est une solution aqueuse tampon.

12. Procédé tel que revendiqué dans l'une quelconque des revendications précédentes, dans lequel, lors de la mesure de l'activité d'enzyme biotinylée ou de l'activité d'inhibiteur d'enzyme biotinylée, se déroule une réaction qui produit une bioluminescence, mesurée quantitativement avec un compteur de photons.

## Patentansprüche

1. Verfahren zur Bestimmung von biologischen Liganden, dadurch **gekennzeichnet,** daß darin die folgenden Stoffe in einem wässrigen Medium nebeneinander bestehen können:
    (A) eine biologisch aktive Verbindung beinhaltend erstens eine immobilisierte Antikörperphase, deren Antikörper fähig ist, mit dem zu messenden Liganden (1) zu reagieren, oder eine immobilisierte Phase eines biologischen Liganden (2) der fähig ist, mit dem Antikörper zu reagieren, und zweitens eine immobilisierte Biotinylenzymphase oder eine immobilisierte Biotinylenzyminhibitorphase,
    (B) eine wasserlösliche Materialkombination von einerseits dem Liganden (2) oder dem Antikörper und andererseits einem Biotinylenzyminhibitor oder einem Biotinylenzym, je nachdem, was in der biologisch aktiven Verbindung nicht enthalten ist, wobei der Biotinylenzyminhibitor dann einer ist, der fähig ist, mit dem Biotinylenzym der biologisch aktiven Verbindung zu reagieren, oder das Biotinylenzym dann eines ist, das fähig ist, mit dem Biotinylenzyminhibitor der biologisch aktiven Verbindung zu reagieren, und
    (C) der zu messende Ligand (1),
    und daß die restliche Biotinylenzymaktivität oder die restliche Biotinylenzyminhibitoraktivität der biologisch aktiven Verbindung oder der wässrigen Lösung gemessen wird und das Ergebnis als Maß des Liganden verwendet wird.

2. Verfahren nach Anspruch 1, wobei Ligand (1) und Ligand (2) ausgewählt werden aus Hormonen, die hergeleitet werden aus endocrinen Drüsen, Plasmaproteinen, viralen Antigenen, Bakterien, α-Fetoproteinen, karzinogenembryonischen Antigenen, Haptenen und ersten Antikörpern in einem doppelten Antikörperverfahren.

3. Verfahren nach Anspruch 1 oder 2, wobei Ligand (1) und Ligand (2) die gleiche Substanz sind.

4. Verfahren nach einem der vorhergehenden Ansprüche, wobei der Antikörper ein Immunoglobulinfragment ist.

5. Verfahren nach einem der Ansprüche 1 bis 3, wobei der Antikörper aus Warmblütlern hergestellt wird.

6. Verfahren nach einem der Ansprüche 1 bis 3, wobei der Antikörper ein monoclonaler Antikörper ist.

7. Verfahren nach einem der vorhergehenden Ansprüche, wobei das Biotinylenzym Propionyl-CoA-Carboxylase, Acetyl-CoA-Carboxylase, Pyruvatcarboxylase, Methyl-CoA-Carboxylase, Methylmalonyl-CoA-Transcarboxylase oder Methylcrotonyl-CoA-Carboxylase ist.

8. Verfahren nach einem der vorhergehenden Ansprüche, wobei der Biotinylenzyminhibitor Avidin, Streptavidin oder Derivate davon sind, die fähig sind, an Biotin zu binden.

9. Verfahren nach einem der vorhergehenden Ansprüche, wobei das Trägermaterial der biologisch aktiven Verbindung ein Einzelblockcopolymerisat oder Propfpolymerisat, zwei polymere Materialien, die aneinander gebunden sind, oder zwei separate Körper zur Immobilisierung der jeweiligen biologisch aktiven Komponente sind.

11

10. Verfahren nach einem der vorhergehenden Ansprüche, wobei die wässrige Lösung einen pH-Wert von 5 bis 8 und eine Temperatur von 15 bis 45°C hat.

11. Verfahren nach Anspruch 10, wobei die wässrige Lösung eine wässrige Pufferlösung ist.

12. Verfahren nach einem der vorhergehenden Ansprüche, bei dem bei der Messung der Biotinylenzymaktivität oder der Biotinylenzyminhibitoraktivität eine Reaktion durchgeführt wird, die Biolumineszenz produziert, die quantitativ unter Verwendung eines Photonenzählers gemessen wird.

FIG. 1.

FIG. 2.

FIG. 3.

FIG. 4.